(19) Europäisches Patentamt
European Patent Office
Office européen des brevets

(11) EP 3 234 551 B1

(12) **FASCICULE DE BREVET EUROPEEN**

(45) Date de publication et mention de la délivrance du brevet:
**25.09.2019 Bulletin 2019/39**

(21) Numéro de dépôt: **15820089.9**

(22) Date de dépôt: **16.12.2015**

(51) Int Cl.:
***G01N 21/00*** *(2006.01)* ***G02B 5/122*** *(2006.01)*
***G01N 21/78*** *(2006.01)*

(86) Numéro de dépôt international:
**PCT/EP2015/080092**

(87) Numéro de publication internationale:
**WO 2016/097052 (23.06.2016 Gazette 2016/25)**

(54) **RETROREFLECTEUR ASSURANT LES FONCTIONS DE RETROREFLEXION ET DE CAPTATION D'UN PARAMETRE DE L'ENVIRONNEMENT**

RETROREFLEKTOREN FÜR RETROFLEXION UND UMGEBUNGSERFASSUNG

RETROREFLECTORS FOR RETROREFLEXION AND ENVIRONMENT SENSING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.12.2014 FR 1462627**

(43) Date de publication de la demande:
**25.10.2017 Bulletin 2017/43**

(73) Titulaire: **Commissariat à l'Énergie Atomique et aux Énergies Alternatives**
**75015 Paris (FR)**

(72) Inventeurs:
- **MARCOUX, Pierre**
  **38120 Saint Egreve (FR)**
- **JOLY, Pierre**
  **38100 Grenoble (FR)**
- **VRIGNAUD, Marjorie**
  **38000 Grenoble (FR)**
- **FATHALLAH, Tarek**
  **38500 Voiron (FR)**

(74) Mandataire: **Brevalex**
**95, rue d'Amsterdam**
**75378 Paris Cedex 8 (FR)**

(56) Documents cités:
**WO-A1-2014/154712**
**US-A1- 2006 088 946**
**US-A1- 2012 140 224**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 234 551 B1

## Description

### DOMAINE TECHNIQUE

La présente invention a trait à un rétroréflecteur réalisé en un matériau spécifique, lequel rétroréflecteur permet à la fois d'assurer les fonctions de rétroréflexion et de captation d'un paramètre de l'environnement ainsi qu'à un procédé de détection d'un paramètre de l'environnement par le biais dudit rétroréflecteur.

L'invention trouve ainsi application dans le domaine de la mesure de paramètres d'environnement, tels que la composition chimique, la température ou la présence de rayonnements, notamment des rayonnements ionisants.

Plus spécifiquement, l'invention peut trouver application dans le domaine du contrôle de l'environnement interne d'endroits clos, tels que les boîtes à gants, les sorbonnes ou encore du contrôle de l'environnement notamment pour la détection de polluants atmosphériques.

L'invention peut également trouver application dans la détection des composés organiques volatils, notamment les composés organiques volatils indicateurs de la présence de bactéries, par exemple, dans des sacs Stomacher, des flacons d'hémoculture, des tests de stérilité.

### ÉTAT DE LA TECHNIQUE ANTÉRIEURE

Les rétroréflecteurs sont des systèmes optiques connus, qui ont pour propriété de renvoyer la lumière dans des directions voisines de celles d'où elle provient, cette propriété étant conservée pour des variations importantes de la direction du faisceau incident. En d'autres termes, grâce aux propriétés du rétroréflecteur, la lumière envoyée par une source sur le rétroréflecteur est renvoyée en direction de celle-ci.

Aussi, vu de la source ou d'un point de vue proche de la source, le rétroréflecteur en fonction présente un aspect particulièrement brillant, d'où la possibilité de les utiliser pour la signification d'objets, par exemple, en tant que catadioptres de véhicules ou en tant que panneaux réfléchissants.

Le retour de la lumière sur elle-même particulièrement efficace avec les rétroréflecteurs peut être également mis à profit pour la mesure de distances très précises par temps de vol, dont l'une des plus connues est celle de la distance terre-lune qui a déjà été évaluée à l'aide de rétroréflecteurs en forme de coin de cube posés sur la lune.

Les propriétés de rétroréflexion peuvent être également utilisées pour la détermination indirecte d'un paramètre de l'environnement, tel que la composition chimique, la température ou la présence d'un rayonnement, notamment un rayonnement ionisant.

Pour ce faire, les rétroréflecteurs sont, actuellement, utilisés selon trois configurations.

Selon une première configuration, comme illustré par le document US 2012/0140224, le rétroréflecteur est utilisé uniquement pour replier le faisceau optique émanant d'une source sur lui-même, afin de mesurer les propriétés d'un matériau sensible à un paramètre de l'environnement situé entre le rétroréflecteur et un instrument, qui constitue à la fois la source lumineuse et un détecteur. Le matériau sensible confronté au paramètre de l'environnement subit une modification de ses propriétés optiques, ce qui va permettre une mesure indirecte du paramètre via des mesures de flux lumineux. Dans cette configuration, la fonction de rétroréflexion et la fonction de captation du paramètre de l'environnement sont remplies par deux éléments distincts l'un de l'autre.

Selon une deuxième configuration, comme illustré par le document US 2007/0036680, le rétroréflecteur comprend, sur ses surfaces réfléchissantes, une couche en un matériau apte à réagir avec l'environnement du rétroréflecteur et dont la réaction modifie les propriétés de réflexion du rétroréflecteur. Aussi, dans cette configuration, il est nécessaire de modifier les surfaces réfléchissantes du rétroréflecteur par le dépôt d'une couche appropriée sensible au paramètre de l'environnement que l'on souhaite mesurer. Du fait que le matériau sensible s'avère confiné au niveau d'une couche déposée sur les surfaces réfléchissantes, la sensibilité se trouve forcément limitée, du fait du volume faible du matériau sensible.

Une troisième configuration consiste à se servir de la dépendance à l'environnement du taux de réflexion au passage d'un milieu à l'autre, qui lui-même varie avec la différence d'indice entre les deux milieux. Ce type de configuration est exposé dans l'article « Proceedings of SPIE-The International Society for Optical Engineering 2839 : 203-214 », dans lequel il est exposé la variation différente de l'indice du verre et de l'eau avec la température. Celle-ci induit notamment une variation du taux de réflexion des surfaces d'un coin de cube de verre plongée dans l'eau pure pour en mesurer sa température. Aussi, pour mesurer le paramètre de l'environnement, qui est ici la température, il est nécessaire de réaliser le rétroréflecteur dans un matériau ayant des propriétés de variation d'indice bien connues et de le placer dans un milieu connu, ici l'eau pure, ayant aussi des propriétés de variation d'indice bien connues et principalement dépendante du paramètre à mesurer. US2012/140224A1, US2006/088946A1, US2003/203212A1, US2006/285116A1 décrivent d'autres exemples d'utilisations de rétroréflecteurs.

WO2014/154712A1 décrit un procédé de fabrication d'un objet moulé de forme complexe. En particulier, un exemple de guide de lumière en matériau poreux est donné pour la détection de gaz.

Au vu de ce qui existe, les auteurs de l'invention se sont donc fixé pour objectif de mettre au point de nouveaux rétroréflecteurs qui permettent d'assurer, outre la rétroréflexion de rayons lumineux, la détection d'un paramètre de l'environnement sans qu'il soit nécessaire,

notamment, d'utiliser deux éléments distincts pour assurer ses deux fonctions ou de recourir au dépôt d'une couche spécifique de captation à la surface du rétroréflecteur.

## EXPOSÉ DE L'INVENTION

Ainsi, l'invention, telle que définie par les revendications attachées, a trait à un rétroréflecteur apte à être placé au contact d'un environnement, comprenant, comme matériau constitutif, un matériau capteur d'un paramètre dudit environnement, ledit matériau induisant une modification des propriétés de transmission optique du rétroréflecteur en présence dudit paramètre et ledit rétroréflecteur étant apte à recevoir un faisceau lumineux incident par une première face et à réémettre un faisceau lumineux par ladite première face (soit en d'autres termes, ledit rétroréflecteur est apte à recevoir un faisceau lumineux incident et à le renvoyer par la même face).

On précise que, par captation d'un paramètre de l'environnement, on entend une détection de la présence de ce paramètre.

On précise que, par matériau constitutif, le matériau constituant le rétroréflecteur, c'est-à-dire le matériau compris entre les faces du rétroréflecteur et délimitant également ces dernières.

Aussi, grâce aux caractéristiques du matériau constitutif du rétroréflecteur de l'invention, les fonctions de rétroréflexion et de captation d'un paramètre de l'environnement sont assurées par le rétroréflecteur en tant que tel, sans qu'il soit nécessaire :

- d'utiliser deux éléments distincts, comme cela est le cas de la première configuration mentionnée ci-dessus, ce qui rend la conception des rétroréflecteurs plus simple ;
- de déposer une couche spécifique de captation à la surface du rétroréflecteur, comme cela est le cas de la deuxième configuration mentionnée ci-dessus.

Selon l'invention, d'un point de vue structural, les rétroréflecteurs de l'invention présentent, avantageusement trois premières faces, dites faces arrières, définissant un trièdre, lesdites trois faces étant convergentes en un point formant un sommet du trièdre, et une quatrième face, dite face avant, opposée audit sommet du trièdre et refermant ledit trièdre, ledit matériau s'étendant entre ces faces.

En d'autres termes, le rétroréflecteur présente trois de ses faces convergeant en point formant un sommet du rétroréflecteur et une quatrième face opposée audit sommet, la forme résultante comprenant, comme matériau constitutif, un matériau de captation d'un paramètre de l'environnement tel que défini ci-dessus.

Plus spécifiquement, les rétroréflecteurs de l'invention peuvent être caractérisés en ce que chaque face arrière susmentionnée s'étend respectivement selon un premier plan, un deuxième plan et un troisième plan, lesdits plans étant sensiblement orthogonaux deux à deux, c'est-à-dire qu'ils forment entre eux un angle compris entre 80° et 100°, de préférence entre 85° et 95°. Avantageusement, avec cette configuration, le faisceau lumineux ressort selon une direction sensiblement parallèle à celle du faisceau lumineux incident.

Encore plus spécifiquement, les rétroréflecteurs peuvent présenter, une forme de coin de cube, c'est-à-dire une forme comprenant trois faces orthogonales deux à deux et une quatrième face refermant le volume délimité par ces trois faces orthogonales et qui présente pour particularité qu'à tout rayon incident correspond un rayon réfléchi de même direction mais de sens opposé.

En particulier, lorsque le rétroréflecteur présente une forme en coin de cube, il y a maximisation de la longueur du trajet optique du faisceau lumineux par rapport à une configuration dite en transmission, dans laquelle le matériau serait disposé entre une source de lumière et le détecteur. En effet, le trajet dans un coin de cube d'épaisseur L (distance entre la face avant et le sommet) est équivalent au trajet rectiligne dans une pastille d'épaisseur 2L. Cela permet d'améliorer la sensibilité de détection.

Il est possible alors de caractériser un milieu contenu dans une enceinte sans rupture de confinement, tel que cela est illustré sur la figure 6 relative à l'exemple 1

Du point de vue du fonctionnement, comme mentionné ci-dessus, du fait de sa fonction de rétroréflexion, le rétroréflecteur est apte à recevoir un faisceau lumineux incident et à le renvoyer par la même face.

Par rapport à la géométrie spécifique mentionnée ci-dessus, le rétroréflecteur est, plus particulièrement, apte à recevoir un faisceau lumineux incident par ladite face avant, ledit faisceau étant centré selon une première direction et est apte à dévier ledit faisceau lumineux lorsque le faisceau lumineux se propage dans ledit matériau, de manière à renvoyer le faisceau lumineux, à travers ladite face avant, selon une deuxième direction parallèle à ladite première direction.

Ce principe de fonctionnement est repris sur la figure 1 jointe en annexe illustrant un rétroréflecteur 1 comprenant 3 faces arrières (numérotées 3) et une face avant (numérotée 5), le faisceau incident et réflechi étant représenté par le numéro 7.

Comme mentionné ci-dessus, au contact d'un environnement dont on veut déterminer un paramètre, le matériau capteur d'un paramètre de l'environnement constitutif du rétroréflecteur est apte à modifier les propriétés de transmission optique du rétroréflecteur en présence dudit paramètre. Plus spécifiquement, le matériau capteur, en présence dudit paramètre, peut induire, avantageusement, une modification de l'intensité lumineuse d'un faisceau lumineux renvoyé par ledit rétroréflecteur en présence dudit paramètre par rapport au cas où le rétroréflecteur n'est pas en présence dudit paramètre. Dans ce cas, d'un point de vue pratique, la modification des propriétés de transmission optique peut être

déterminée par la mesure du rapport entre l'intensité lumineuse du faisceau incident et l'intensité lumineuse du faisceau renvoyé par le rétroréflecteur.

D'une façon générale, le matériau capteur doit être poreux ou perméable ou apte à changer d'aspect sous l'effet de la température ou d'un rayonnement.

Par changer d'aspect, on entend une modification de la propriété de transmission optique apte à être modifiée, dans une plage de longueur d'onde donnée, lorsque le rétroréflecteur est placé au contact d'un paramètre d'un environnement (par exemple, présence d'un gaz, température, rayonnement ionisant, par exemple, un rayonnement gamma ou un rayonnement X).

Ainsi, le paramètre qu'il s'agisse d'une molécule, d'une température ou d'un rayonnement pénètre dans le matériau, induisant une variation de la transmission optique.

Le matériau capteur d'un paramètre de l'environnement est, avantageusement, un matériau poreux et, plus spécifiquement, un matériau à porosité ouverte, c'est-à-dire une porosité permettant une communication entre les différents pores.

Avantageusement, le matériau capteur est également un matériau transparent.

Ces caractéristiques de porosité trouvent, tout particulièrement, leur importance, lorsque le paramètre de l'environnement à capter est un gaz, et plus, spécifiquement, un composé organique volatil. La porosité permet ainsi au gaz de pénétrer dans le volume du rétroréflecteur, afin d'en modifier la transmission après captation de ce gaz par le matériau capteur constitutif du rétroréflecteur.

Avantageusement, la porosité définie comme la fraction du volume libre relativement au volume total du matériau (c'est-à-dire le rapport (volume libre/volume total)) est comprise entre 2% et 80%, cette porosité étant déterminée par mesure d'adsorption-désorption d'azote à 77K.

Avantageusement, le matériau capteur peut présenter une surface spécifique allant de 100 à 5000 $m^2/g$.

Lorsque le matériau capteur du rétroréflecteur est un matériau poreux destiné à la captation d'un gaz (qui constitue le paramètre de l'environnement), le matériau constitutif présente, avantageusement, un indice de réfraction élevé pour compenser la perte de valeur de l'indice de réfraction du matériau poreux lié au fait que les pores remplis de gaz présentent un indice de réfraction proche de 1, ce qui fait diminuer l'indice de réfraction du matériau poreux.

Indépendamment de la porosité du matériau, le matériau du rétroréflecteur peut présenter, avantageusement, un indice de réfraction allant de 1,2 à 2, cet indice de réfraction pouvant être mesuré par une méthode impliquant un microscope, comme exposé dans la partie d) de l'exemple 1.

Lorsque le matériau capteur du rétroréflecteur est un matériau poreux destiné à la captation d'un liquide, tel qu'un environnement aqueux d'indice 1,33, (qui constitue le paramètre de l'environnement), le matériau capteur peut présenter, avantageusement, un indice de réfraction allant de 1,66 à 2,7, cet indice de réfraction pouvant être mesuré par une méthode impliquant un microscope, comme exposé dans la partie d) de l'exemple 1.

Le ratio entre l'indice du matériau et l'indice du milieu à caractériser est compris entre 1,2 et 2. Dans le cas d'un gaz, l'indice du matériau est, avantageusement, compris entre 1,2 et 2, car l'indice du milieu est 1. On peut définir l'indice du matériau sur l'indice du milieu à caractériser comme étant un indice relatif.

Plus le rapport d'indice est élevé, plus la direction du faisceau réfléchi et incident peut s'écarter de la normale à la face avant du rétroréflecteur, cette dernière étant la face traversée par le faisceau incident et le faisceau réfléchi.

Du point de vue de sa composition, le matériau capteur d'un paramètre de l'environnement peut être en un matériau organique ou inorganique, de préférence, formant matrice, comprenant, en son sein, un ou plusieurs composés, généralement organiques, porteurs d'un ou plusieurs groupes remplissant une fonction de captation d'un paramètre de l'environnement, cette formulation couvrant deux alternatives envisageables :

- une première alternative où le ou les composés porteurs d'un ou plusieurs groupes sont inclus dans le matériau organique ou inorganique sans être liés de manière covalente à ce dernier ;
- une deuxième alternative où le ou les composés porteurs d'un ou plusieurs groupes sont liés, de manière covalente, audit matériau organique ou inorganique.

Selon cette deuxième alternative, lorsque le ou les composés porteurs d'un ou plusieurs groupes sont liés, de manière covalente, à un matériau inorganique, le matériau résultant constitue un matériau hybride inorganique-organique.

Indépendamment de l'alternative susmentionnée :

- lorsque le matériau formant matrice est un matériau organique, il peut s'agir d'un matériau en au moins un polymère, par exemple, un matériau en polydiméthysiloxane (connu sous l'abréviation PDMS), qui présente la capacité d'être perméable aux gaz et la capacité à être façonné par moulage ; et
- lorsque le matériau formant matrice est un matériau inorganique, il peut s'agir d'un matériau du type oxyde(s) inorganique(s) et, avantageusement, un matériau du type oxyde(s) d'un élément métallique et/ou d'un élément métalloïde (tel que Si).

Avantageusement, le matériau du type oxyde(s) inorganique(s) comprend un ou plusieurs oxydes d'un élément choisi parmi le silicium, le titane, le zirconium, l'aluminium, le vanadium, le chrome, l'yttrium, le

tungstène, le niobium, le molybdène.

De manière encore plus spécifique, le matériau du type oxyde(s) inorganique(s) peut comprendre de la silice et un oxyde présentant un indice de réfraction plus élevée que la silice, cet oxyde pouvant être choisi parmi la zircone, l'oxyde de titane.

Ce matériau du type oxyde(s) inorganique(s) peut être, avantageusement, un matériau sol-gel.

Par « matériau sol-gel », on entend, classiquement, un matériau obtenu par un procédé sol-gel consistant à utiliser comme précurseurs, par exemple, des alcoxydes, identiques ou différents, de formule $M(OR)_n(R')_m$, dans laquelle M est un élément chimique tel que le silicium (Si), le titane (Ti), le zirconium (Zr), le vanadium (V), le tungstène (W), le molybdène (Mo), le chrome (Cr), le niobium (Nb), l'aluminium (Al), l'yttrium (Y), R et R' représentent un groupement alkyle, n étant un entier positif et m étant égal à 0 ou étant un entier positive, la somme (m+n) correspondant au degré de valence de M.

Les matériaux sol-gel sont généralement préparés dans un solvant, qui est de préférence miscible à l'eau et évaporable progressivement et dans des conditions douces, dans lequel les précurseurs sont solubles.

Dans le cas des alcoxydes de silicium, on peut notamment citer, comme solvant, les alcools, tels que le méthanol, l'éthanol ; les éthers, tels que le diéthyléther et le tétrahydrofurane ; les solvants chlorés, tels que le chloroforme, $CH_2Cl_2$, $C_2H_5Cl_2$, d'autres solvants aprotiques comme l'acétonitrile, l'acétone, la méthyléthylcétone, ou le dioxane ou d'autres solvants protiques comme l'acide acétique, le formamide.

En présence d'eau, l'hydrolyse des groupements alcoxyde (-OR) intervient et ces derniers sont transformés en groupements silanol (M-OH) qui se condensent en formant des groupements siloxane (M-O-M). De petites particules de taille généralement inférieure à 1 nanomètre sont alors formées. Elles s'agrègent et forment des amas lacunaires en suspension dans le liquide : c'est le sol. La polycondensation se poursuivant au cours du temps, la viscosité du sol augmente jusqu'à gélification : le sol devient un gel.

Un matériau sol-gel solide est ensuite obtenu par séchage du gel, appelé xérogel. Au cours de cette étape, les solvants résiduels et interstitiels s'échappent du réseau polymérique formé et s'évaporent, ce qui provoque la contraction du matériau, notamment d'un facteur 2 sur chacune des dimensions par rapport au sol. On obtient donc un matériau final dont le volume est réduit par comparaison au volume occupé par le sol.

Les matériaux sol-gel peuvent être classés en fonction de la taille des pores. En effet selon les règles établies par l'International Union of Pure & Applied Chemistry (IUPAC) on peut distinguer, selon le diamètre moyen des pores dans un matériau, les micropores (moins de 20 Å), les mésopores (20-500 Å) et les macropores (plus de 500 Â).

Comme mentionné ci-dessous, le matériau capteur d'un paramètre de l'environnement peut être un matériau organique ou inorganique, de préférence, formant matrice, comprenant, en son sein, un ou plusieurs composés, généralement organiques, porteurs d'un ou plusieurs groupes remplissant une fonction de captation d'un paramètre de l'environnement (dit plus loin groupe spécifique), ce paramètre de l'environnement pouvant être :

- la composition chimique de l'environnement, par exemple, la présence dans l'environnement de composés chimiques, tels que des polluants atmosphériques, des composés organiques volatils, connus sous la dénomination « COV » ;
- la température de l'environnement ;
- la présence de rayonnement dans l'environnement, en particulier, un rayonnement ionisant, par exemple, un rayonnement X ou un rayonnement y.

Pour ces deux derniers paramètres, la détection de la température et du rayonnement peut être intrinsèque au matériau, sans qu'il soit nécessaire d'y ajouter des capteurs spécifiques.

Plus spécifiquement, le mécanisme de captation du paramètre de l'environnement par le ou les groupes susmentionnés peut répondre aux schémas suivants :

- une captation du paramètre de l'environnement par modification chimique du ou des groupes spécifiques, ce qui peut être le cas lorsque le paramètre est un composé chimique qui réagit chimiquement avec le ou les groupes susmentionnés ou encore lorsque le paramètre est un paramètre physique, tel que la température ou des rayonnements, qui peut induire une modification chimique du ou des groupes susmentionnés;
- une captation du paramètre de l'environnement par affinité chimique entre le ou les groupes spécifiques et le paramètre de l'environnement, ce qui peut être le cas lorsque le paramètre de l'environnement est un composé chimique présentant une affinité chimique avec le ou les groupes susmentionnés (par exemple, d'un point de vue de l'hydrophilie ou de l'hydrophobicité) par exemple, par établissement de liaisons faibles entre le ou les groupes susmentionnés et le composé chimique sans que cela n'induise de modifications chimiques du ou des groupes susmentionnés et du composé chimique ; et
- une combinaison d'une captation du paramètre par modification chimique du ou des groupes spécifiques du composé organique et d'une captation du paramètre par affinité chimique entre le ou les groupes spécifiques du composé organique et le composé chimique.

En d'autres termes, lorsque le paramètre de l'environnement est un composé chimique, le ou les composés, généralement organiques, porteurs d'un ou plusieurs groupes remplissant une fonction de captation d'un paramètre de l'environnement peuvent présenter un ou plusieurs groupes aptes à capter ledit composé chimique avec modification chimique de celui-ci et/ou aptes à capter ledit composé chimique par affinité chimique sans modification chimique du composé chimique.

Quel que soit le mécanisme mis en jeu, le matériau du rétroréflecteur, au contact du paramètre de l'environnement que l'on souhaite déterminer (présence d'un gaz, rayonnement ionisant, tel qu'un rayonnement γ ou un rayonnement X), peut être amené à changer de couleur, c'est-à-dire à subir une modification de ses propriétés de transmission optique dans une plage de longueurs d'onde donnée.

Plus spécifiquement, lorsque le paramètre de l'environnement est un composé chimique apte à modifier chimiquement le ou les composés, généralement organiques, compris dans le matériau, cette modification chimique peut résulter d'une réaction acido-basique entre le composé chimique et le ou les groupes spécifiques du ou des composés, généralement organiques, compris dans le matériau. Ce ou ces groupes spécifiques peuvent être un groupe attracteur de protons, lorsque le composé chimique comprend un groupe donneur de protons, ou peuvent être un groupe donneur de protons, lorsque le composé chimique comprend un groupe attracteur de protons.

Cette modification chimique peut également résulter d'une réaction covalente entre le composé chimique et le ou les groupes spécifiques compris au sein du matériau, auquel cas il résulte une liaison covalente entre ledit composé chimique et le ou les groupes spécifiques. Le composé organique comprenant au moins un tel groupe spécifique au sein du matériau peut être ainsi qualifié de molécule sonde.

Plus spécifiquement, lorsque le paramètre de l'environnement est un composé chimique qui présente une affinité chimique pour le ou les composés, généralement organiques, compris dans le matériau, le ou ou groupes spécifiques du ou des composés, généralement organiques, compris dans le matériau peuvent être :

- des groupes hydrophobes, lorsque le composé chimique comprend un groupe hydrophobe, par exemple, tel qu'un groupe hydrocarboné ; et
- des groupes hydrophiles, lorsque le composé chimique comprend un groupe hydrophile, par exemple, tel qu'un groupe OH.

Comme mentionné ci-dessus, il peut y avoir à la fois une combinaison d'une captation du paramètre par modification chimique du ou des groupes spécifiques du composé organique et d'une captation du paramètre par affinité chimique avec le ou les groupes spécifiques du composé organique.

Dans ce cas, le composé compris au sein du matériau est, avantageusement, porteur d'au moins un groupe remplissant une fonction de captation d'un paramètre de l'environnement par modification chimique dudit groupe par le paramètre de l'environnement (tel qu'un composé chimique) et au moins un groupe remplissant une fonction de captation d'un paramètre de l'environnement par affinité chimique du paramètre de l'environnement (tel qu'un composé chimique) avec ledit groupe.

Aussi, le composé compris au sein du matériau peut comprendre :

- un groupe hydrocarboné, tel qu'un groupe alkylène, lequel remplit, classiquement, une fonction hydrophobe, ce qui peut permettre d'attirer, par affinité chimique, un composé chimique hydrophobe ; et
- un groupe attracteur de protons, tel qu'un groupe amine, lequel va être capable de réagir chimiquement avec un proton du même composé chimique.

Un tel composé organique peut être représenté, lorsqu'il est lié de manière covalente au matériau du type oxyde inorganique ou organique, par la formule suivante :

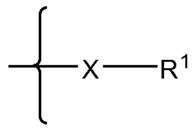

dans laquelle :

- X représente un groupe hydrocarboné du type alkylène ; et
- $R^1$ représente un groupe attracteur de protons, tel qu'un groupe amine primaire.

l'accolade indiquant l'endroit par lequel le composé est lié, de manière covalente, au matériau du type oxyde inorganique ou organique.

Indépendamment du mécanisme de captation, le phénomène de captation du paramètre de l'environnement, du fait qu'il se produit au coeur du matériau, induit une modification des propriétés de transmission optique du matériau constitutif du rétroréflecteur dans son volume, ce changement de propriété de transmission optique étant ensuite utilisé pour permettre une mesure indirecte du paramètre concerné via des mesures de flux lumineux.

Selon un mode particulier de réalisation de l'invention, les rétroréflecteurs de l'invention peuvent être des rétroréflecteurs dont le matériau capteur comprend un matériau comprenant de la silice et au moins un autre oxyde d'un élément choisi parmi le titane, le zirconium, l'aluminium, le vanadium, le chrome, l'yttrium, le tungstène, le niobium, le molybdène, une partie des atomes de silicium étant liée, de manière covalente à un composé organique porteur d'un ou plusieurs groupes remplissant une fonction de captation d'un paramètre de l'environnement.

Encore plus spécifiquement, selon ce mode particulier de réalisation, les rétroréflecteurs de l'invention peuvent être des rétroréflecteurs dont le matériau capteur comprend de la silice et au moins un autre oxyde d'un élément choisi parmi le zirconium, le titane.

Encore plus spécifiquement, selon ce mode particulier de réalisation, le composé organique porteur d'un ou plusieurs groupes remplissant une fonction de captation d'un paramètre de l'environnement répond à la formule suivante :

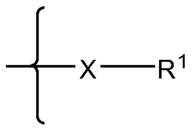

dans laquelle :

- X représente un groupe hydrocarboné du type alkylène ; et
- $R^1$ représente un groupe attracteur de protons, tel qu'un groupe amine primaire.

l'accolade indiquant l'endroit par lequel le composé est lié, de manière covalente, à une partie des atomes de silicium de la silice.

Pour le mode de réalisation mentionné ci-dessus, les rétroréflecteurs sont particulièrement adaptés pour la détection d'un composé organique volatil présentant un caractère acide, tel que l'orthonitrophénol.

A titre alternatif, on peut citer, comme composé organique porteur d'un ou plusieurs groupes remplissant une fonction de captation d'un paramètre de l'environnement, les composés suivants :

- des composés appartenant à la catégorie des indicateurs colorés de pH, tels que le bleu de bromothymol, le bleu de bromophénol, le bleu de thymol, le bleu de bromothymol étant notamment apte à détecter l'acide carbonique ;
- des composés appartenant à la catégorie des sondes solvatochromiques, c'est-à-dire des composés qui changent de couleur en fonction de la polarité du milieu dans lequel ils se trouvent, tels que le vert de malachite, le Brilliant Yellow, le Reichardt's Dye (ou colorant de Reichardt apte à détecter des molécules polaires, telles que l'acétonitrile et l'acide cyanhydrique) ;
- des composés organiques appartenant à la famille des aldéhydes, tels que le diméthylaminocinnamaldéhyde pour la détection de l'indole et de la β-naphthylamine ;
- des composés organiques appartenant à la famille des disulfures, tels que le 5,5'-dithiobis-(acide 2-nitrobenzoïque) pour la détection des composés sulfures ou encore l'acide dithionitrobenzoïque pour la détection de $H_2S$ ;
- des composés organiques appartenant à la famille des amines primaires, telles que le 3-aminopropyle pour la détection des nitrophénols ;
- des composés organiques appartenant à la famille des acides carboxyliques, tels que l'acide isovalérique ; et
- des composés organiques appartenant à la famille des cétones, telles que la 4-amino-3-pentèn-2-one pour la détection du formaldéhyde.

Ces différents composés peuvent être notamment utilisés pour la détermination d'un paramètre du type composé organique volatil, ces composés pouvant remplir le rôle de facteur de révélation, qui assurent une modification de l'absorbance du rétroréflecteur, dans lequel ils sont contenus.

Les rétroréflecteurs de l'invention sont destinés, comme leur nom l'indique, à réfléchir une lumière incidente tout en permettant la détection d'un paramètre de l'environnement, tel qu'un composé organique volatil présent dans l'environnement grâce à la capacité du matériau capteur de modifier les propriétés de transmission optique du rétroréflecteur, et plus spécifiquement, par exemple, d'induire une modification de l'intensité lumineuse d'un faisceau lumineux renvoyé par ledit rétroréflecteur en présence dudit paramètre de l'environnement par rapport à l'intensité lumineuse du faisceau lumineux incident.

Aussi l'invention a également trait à un procédé de détection d'au moins un paramètre de l'environnement comprenant les étapes suivantes :

- une étape de mise en contact d'un rétroréflecteur de l'invention avec un environnement, dont on veut détecter un paramètre donné ;
- une étape d'éclairage du rétroréflecteur par un premier faisceau lumineux présentant une première longueur d'onde, ladite première longueur d'onde correspondant à une longueur d'onde susceptible de provoquer une variation de la transmission optique du matériau constituant le rétroréflecteur après captation du paramètre de l'environnement ;
- une étape d'analyse d'un premier faisceau lumineux réfléchi par le rétroréflecteur, de laquelle l'on en déduit, le cas échéant, la présence dudit paramètre de l'environnement.

Ledit premier faisceau lumineux peut comporter une pluralité de longueurs d'onde et l'étape d'analyse du faisceau réfléchi peut comprendre une analyse dudit faisceau à au moins deux longueurs d'onde de ladite pluralité de longueurs d'onde.

En outre, le procédé peut comprendre également les étapes suivantes :

- une deuxième étape d'éclairage du rétroréflecteur par le faisceau lumineux comprenant une deuxième

longueur d'onde, ladite deuxième longueur d'onde correspondant à une longueur d'onde susceptible de provoquer une deuxième variation de la transmission optique du matériau constituant le rétroréflecteur après captation du paramètre de l'environnement, ladite deuxième variation étant moindre que ladite première variation ;

- une étape d'analyse d'un deuxième faisceau lumineux réfléchi par le rétroréflecteur ;
- une étape de comparaison desdits premier et deuxième faisceaux réfléchis, de laquelle l'on en déduit, le cas échéant, la présence du paramètre de l'environnement.

Par exemple, lorsque le matériau capteur du paramètre de l'environnement est apte, en présence du paramètre de l'environnement que l'on veut détecter, à modifier l'intensité lumineuse du faisceau lumineux réfléchi par rapport à celle du faisceau lumineux incident, l'étape d'analyse peut consister, dans un premier temps, à mesurer l'intensité lumineuse du faisceau réfléchi puis, dans un deuxième temps, à la comparer à l'intensité du faisceau lumineux de l'étape d'éclairage, de sorte à faire la corrélation avec la présence du paramètre de l'environnement.

A titre d'exemple, pour la détection d'un paramètre de l'environnement, tel qu'un COV spécifique, le rétroréflecteur est éclairé, lors de l'étape d'éclairage, par un dispositif d'éclairage, par exemple, une ou plusieurs diodes électroluminescentes (LED), émettant des rayons incidents à au moins une longueur d'onde apte à être absorbée par le composé organique volatil dont on veut détecter l'éventuelle présence et éventuellement émettant des rayons incidents à au moins une autre longueur faisant office de longueur d'onde de référence. Un dispositif de mesure, telle qu'une caméra couleur, assure la mesure de l'intensité des rayons aux longueurs d'onde susmentionnées réfléchis par le rétroréflecteur. Aussi, la détection du composé organique volatil capturé par le rétroréflecteur peut être matérialisée, lors de l'étape d'analyse, par une diminution de l'intensité du rayon réfléchi présentant la longueur d'onde d'absorption du composé organique volatil, tandis que l'intensité lumineuse du rayon réfléchi présentant la longueur d'onde de référence reste constante.

Plus spécifiquement, lorsque le réfroréflecteur a pour visée de détecter un composé organique volatil émis par une bactérie suite à l'hydrolyse d'un substrat enzymatique donné, la détection en tant que telle peut être réalisée après incubation sur un modèle similaire à celui exposé au paragraphe précédent (c'est-à-dire avec l'utilisation de deux longueurs d'onde : une longueur d'onde spécifique et une longueur d'onde de référence) ou par un suivi cinétique, c'est-à-dire un suivi à une seule longueur d'onde spécifique avant et après l'introduction du substrat enzymatique. De manière pratique, les rétroréflecteurs de l'invention peuvent être utilisés pour la détection de bactéries dans un liquide, par exemple un liquide corporel, et notamment du sang, en disposant le rétroréflecteur dans un bouchon, de telle sorte que la face principale soit accessible depuis l'extérieur. On peut alors détecter la présence d'o-nitrophénol entre le liquide et le rétroréflecteur, ce qui traduit la présence d'un microorganisme dans le liquide corporel.

Plus généralement, cette application peut être transposée à tout produit, solide ou liquide, dont on souhaite contrôler la stérilité.

Lorsque le rétroréflecteur a pour visée de détecter un paramètre de l'environnement d'un volume (par exemple, un volume contenu dans une enceinte) dont veut connaître les caractéristiques, le rétroréflecteur peut être placé sur la face interne de la paroi du volume, étant entendu que la paroi doit être transparente à la lumière utilisée pour interroger le rétroréflecteur. On peut ainsi avoir l'avantage de placer toute l'instrumentation de lecture du rétroréflecteur (émission et détection du faisceau lumineux) devant la face principale du rétroréflecteur et non de part et d'autre de ce dernier.

Lorsque le rétroréflecteur comprend un matériau capteur comprenant un matériau du type oxyde(s) inorganique(s), il peut être préparé par un procédé par voie sol-gel comprenant les étapes suivantes :

a) une étape de remplissage de la cavité interne d'un moule, ladite cavité interne ayant une forme correspondant à celle du rétroréflecteur que l'on souhaite obtenir par une solution sol-gel ;
b) une étape de gélification de la solution sol-gel dans le moule ; et
c) une étape de séchage dans ledit moule du gel obtenu en b), moyennant quoi ledit gel se transforme en le matériau constitutif du rétroréflecteur.

Des détails concernant la mise en oeuvre de ces étapes peuvent être retrouvés dans la demande FR 2 980 789, incorporée ici par référence.

Comme mentionné ci-dessus, il est introduit, dans la cavité interne jusqu'à remplissage complet de cette dernière, une solution sol-gel.

Cette solution sol-gel peut être également préparée préalablement à l'étape a).

Cette étape de préparation peut consister à mettre en contact un ou plusieurs précurseurs moléculaires d'un élément métallique et/ou d'un élément métalloïde, un composé généralement organique porteur d'un ou plusieurs groupes remplissant une fonction de captation d'un paramètre de l'environnement ou un précurseur de celui-ci et éventuellement d'autres adjuvants, tel que de l'eau, un agent complexant de l'un au moins desdits précurseurs, avec un milieu comprenant un ou plusieurs solvants organiques.

Les précurseurs moléculaires d'élément métallique et/ou d'élément métalloïde peuvent se présenter, plus généralement sous forme de composés organométalliques d'élément métallique et/ou d'élément métalloïde, tels que, notamment, des alcoxydes, par exemple,

ceux répondant à la formule $(R^2O)_nM$ ou $R^3M(OR^4)_{n-1}$, dans laquelle M désigne l'élément métallique et/ou l'élément métalloïde, n représente le degré d'oxydation de M et $R^2$, $R^3$ et $R^4$ représentent, indépendamment l'un de l'autre, un groupe alkyle, linéaire ou ramifié, pouvant comporter de 1 à 10 atomes de carbone ou un groupe phényle.

Le composé généralement organique porteur d'un ou plusieurs groupes remplissant une fonction de captation d'un paramètre de l'environnement, comme mentionné ci-dessus, peut être compris au sein du matériau sans qu'il n'y ait de liaisons covalentes entre l'oxyde inorganique (premier cas) et ledit composé ou peut être lié, de manière covalente, audit oxyde inorganique (second cas).

Selon le premier cas, le composé généralement organique susmentionné peut être inclus directement dans la solution sol-gel au cours de son procédé de préparation, ce composé ne réagissant lors de l'étape b) avec les précurseurs susmentionnés. Après gélification de la solution sol-gel, il se retrouve directement piégé par encapsulation au sein du gel, sans qu'il n'y ait de liaison de covalence entre le composé et les unités constitutives du gel.

Selon le second cas, le composé généralement organique susmentionné peut être introduit, lors de la préparation de la solution sol-gel, directement *via* un précurseur de formule $R^5M(OR^6)_{n-1}$ avec M étant tel que défini ci-dessus, $R^6$ réprésentant un groupe alkyle, linéaire ou ramifié, pouvant comporter de 1 à 10 atomes de carbone ou un groupe phényle, $R^5$ correspondant au composé organique et n correspondant au degré de valence de M. Lors de l'étape de gélification de la solution sol-gel, le précurseur de formule $R^5M(OR^6)_{n-1}$ réagit avec les autres précurseurs susmentionnés pour former un réseau d'oxyde incorporant l'élément M du précurseur de formule $R^5M(OR^6)_{n-1}$, cet élément M se trouvant, dans le réseau, lié de manière covalente au groupe $R^5$, qui est le composé organique.

A titre d'exemple, lorsque M correspond à l'élément silicium, on peut citer, comme précurseur, le 3-aminopropyltriéthoxysilane (APTES, $Si(C_3H_6NH_2)(OC_2H_5)_3$), le 3-aminobutyltriéthoxysilane (ABTES, $Si(C_4H_8NH_2)(OC_2H_5)_3$), le 3-aminopropyltriméthoxysilane (APTMS, $Si(C_3H_6NH_2)(OCH_3)_3$), le (3-(méthylamino)propyl)triméthoxysilane ($Si(C_3H_6NHCH_3)(OCH_3)_3$), le 3-carboxypropyltriéthoxysilane ($Si(C_3H_6CO_2H)(OC_2H_5)_3$), le 3-carboxypropyltriméthoxysilane ($Si(C_3H_6CO_2H)(OCH_3)_3$), le 1,2-bis(triéthoxysilyl)éthane ($(OC_2H_5)_3Si-CH_2-CH_2-Si(OC_2H_5)_3$), le 1,2-bis(triméthoxysilyl)éthane ($(OCH_3)_3Si-CH_2-CH_2-Si(OCH_3)_3$), le (3,3,3-trichloropropyl)triéthoxysilane ($Si(C_2H_5CCl_3)(OC_2H_5)_3$) et le 3,3,3-trifluoropropyl-triméthoxysilane ($Si(C_2H_5CF_3)(OCH_3)_3$) et leurs mélanges.

De préférence, le solvant est un solvant organique choisi parmi :

*les monoalcools aliphatiques ou aromatiques, saturés ou insaturés, par exemple ceux de formule R"-OH, dans laquelle R' représente un groupe alkyle, linéaire ou ramifié, comprenant de 1 à 30 atomes de carbone, de préférence de 1 à 10 atomes de carbone ou un groupe phényle ;
*les diols, par exemple, ceux de formule HO-R'''-OH, dans laquelle R''' représente un groupe alkylène, linéaire ou ramifié, comprenant de 1 à 30 atomes de carbone, de préférence de 1 à 10 atomes de carbone, ou un groupe phénylène.

A titre d'exemples de diols, on peut citer l'éthylèneglycol, le diéthylèneglycol ou encore le triéthylèneglycol.

Outre la présence d'un ou plusieurs précurseurs moléculaires et d'un ou plusieurs solvants organiques tels que définis ci-dessus, la solution sol-gel peut comprendre d'autres adjuvants, tels que :

- l'eau, qui peut contribuer à faciliter le processus de gélification de la solution sol-gel ;
- des catalyseurs permettant d'accélérer la cinétique des réactions d'hydrolyse et de condensation lors de la transformation de la solution sol-gel en gel (ces catalyseurs pouvant être un acide inorganique, tel que l'acide chlorhydrique, un acide organique, tel que l'acide acétique) ;
- des agents complexants de l'un au moins desdits précurseurs, en vue par exemple, de ralentir l'hydrolyse de certains précurseurs, comme les précurseurs à base de zirconium ou à base de titane qui s'hydrolysent très rapidement en raison de la charge partielle positive importante sur l'élément métallique.

A titre d'exemples d'agents complexants, on peut citer l'acide acétique, l'acétylacétone ou le 2-méthoxyéthanol.

A titre d'exemple, lorsque le rétroréflecteur comprend, comme matériau constitutif, un matériau comprenant de la silice et au moins un autre oxyde d'un élément choisi parmi le zirconium, le titane et comprend, comme composé organique porteur d'un ou plusieurs groupes remplissant une fonction de captation d'un paramètre de l'environnement, un composé répondant à la formule suivante :

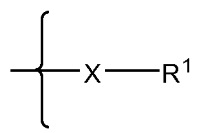

dans laquelle :

- X représente un groupe hydrocarboné du type alkylène ; et
- $R^1$ représente un groupe attracteur de protons, tel

qu'un groupe amine primaire.

l'accolade indiquant l'endroit par lequel le composé est lié, de manière covalente, à une partie des atomes de silicium de la silice, le procédé de préparation de la solution sol-gel peut comprendre les étapes suivantes :

d) une étape de mise en contact d'un alcoxysilane de formule $(R^2O)_4Si$ ou $R^3Si(OR^4)_3$ avec $R^2$, $R^3$ et $R^4$ étant tels que définis ci-dessus, avec un solvant organique et éventuellement de l'eau, moyennant quoi l'on obtient une première solution ;
e) une étape de mise en contact d'un alcoxysilane de formule $(R^2O)_nM$ ou $R^3M(OR^4)_{n-1}$ avec M étant Ti ou Zr et $R^2$, $R^3$, $R^4$ et n étant tels que définis ci-dessus avec un agent complexant de M, de préférence, choisi parmi l'acide acétique, l'acétylacétone ou le 2-méthoxyéthanol, moyennant quoi l'on obtient une deuxième solution ;
f) une étape de mise en contact de ladite première solution et de ladite deuxième solution ;
g) une étape d'ajout au mélange résultant de l'étape c) d'un alcoxysilane de formule $R^5Si(OR^6)_3$ avec $R^6$ étant tel que défini ci-dessus et $R^5$ correspondant à la formule :

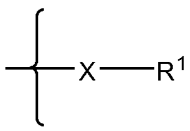

X et $R^1$ étant tels que définis ci-dessus.

La solution sol-gel obtenue à l'issue de l'étape d) est ensuite utilisée directement pour la mise en oeuvre du procédé de préparation d'un rétroréflecteur tel que défini ci-dessus.

Lorsque le rétroréflecteur comprend un matériau capteur comprenant un matériau organique du type polymère et un ou plusieurs composés, généralement organiques, porteurs d'une ou plusieurs fonctions capteurs, ledit ou lesdits composés n'étant pas liés de manière covalente au matériau organique, il peut être préparé selon les procédés suivants :

- un procédé impliquant une étape de polymérisation, dans un moule dont la forme correspond à la forme du rétroréflecteur, d'un mélange comprenant un ou plusieurs monomères et le ou les composés, généralement organiques, porteurs d'un ou plusieurs groupes remplissant la fonction de captation du paramètre de l'environnement (dit ci-dessous première variante) ;
- un procédé impliquant une étape de polymérisation, dans un moule dont la forme correspond à la forme du rétroréflecteur, d'un mélange comprenant un ou plusieurs monomères précurseurs dudit polymère suivie d'une étape de dopage du polymère obtenu par le ou les composés, généralement organiques, porteurs d'un ou plusieurs groupes remplissant la fonction de captation du paramètre de l'environnement (dit ci-dessous seconde variante).

La première variante est particulièrement adaptée pour les matériaux polymérisables susceptibles d'être obtenus par polymérisation à température ambiante, une telle température permettant d'éviter la dégradation du ou des composés susmentionnés.

La seconde variante est particulièrement adaptée pour les matériaux polymères poreux, dans lequel il est possible, après polymérisation, d'incorporer par dopage le ou les composés susmentionnés (par exemple, des molécules sondes aptes à modifier la transmission dans une plage de longueur d'ondes donnée), par exemple, selon une quantité pouvant être inférieure à 10% massique.

Plus spécifiquement, on peut citer, comme matériau polymère, un matériau polydiméthysiloxane (dénommé également PDMS), présentant la propriété d'être perméable aux gaz. Du fait de sa perméabilité aux gaz, il est possible d'incorporer le ou les composés organiques porteurs d'un ou plusieurs groupes remplissant la fonction de captation par imprégnation sous forme gazeuse du PDMS préalablement polymérisé à hautes températures.

D'autres caractéristiques et avantages de l'invention apparaîtront du complément de description qui suit qui se rapporte à un exemple de préparation d'un rétroréflecteur conformément au procédé de l'invention.

Bien entendu, ce complément de description n'est donné qu'à titre d'illustration de l'invention et n'en constitue en aucun cas une limitation.

## BREVE DESCRIPTION DES FIGURES

La figure 1 représente une vue en coupe transversal d'un rétroréflecteur conforme à l'invention présentant une forme de coin de cube.

La figure 2 représente une photographie en vue de dessus du rétroréflecteur obtenu à l'exemple 1 (partie b) et de son modèle (partie a).

La figure 3 est un schéma illustrant le principe de mesure de l'indice de réfraction au moyen d'un microscope.

La figure 4 est un schéma illustrant le montage expérimental permettant de mesurer l'intensité de la lumière réfléchie par un rétroréflecteur.

La figure 5 représente une photographie de la lumière réfléchie par le rétroréflecteur obtenu à l'exemple 1.

La figure 6 représente un flacon dont on souhaite caractériser l'environnement intérieur à l'aide du rétroréflecteur préparé à l'exemple 1 placé dans un

septum refermant ledit flacon.

La figure 7 est un graphique illustrant l'évolution de l'intensité I à 415 nm et à 590 nm en fonction du temps t (en minutes) pour le rétroréflecteur de l'exemple 1 en présence d'orthonitrophénol.

## EXPOSÉ DÉTAILLÉ DE MODES DE RÉALISATION PARTICULIERS

EXEMPLE 1

Cet exemple illustre la préparation par voie sol-gel d'un rétroréflecteur conforme à l'invention, ce rétroréflecteur présentant une forme de coin de cube ou plus spécifiquement une forme comprenant trois faces planes perpendiculaires l'une par rapport à l'autre et une face curviligne reliant les sommets des faces perpendiculaires.

Pour ce faire, la préparation du rétroréflecteur se déroule en trois étapes :

- une étape de préparation du moule (dite ci-dessous étape a) ;
- une étape de préparation de la solution sol-gel (dite ci-dessous étape b) ;
- une étape de fabrication du rétroréflecteur en tant que tel (dite ci-dessous étape c).

Enfin, cet exemple comporte une partie relative à la caractérisation du xérogel constitutif du rétroréflecteur (dite ci-dessous étape d).

a) Etape de préparation du moule

Le modèle utilisé pour la fabrication du moule est illustré par la partie a) de la figure 2, lequel se présente sous une forme comprenant deux faces planes perpendiculaires l'une par rapport à l'autre et une troisième face curviligne reliant les sommets des deux faces perpendiculaires.

Le moule est préparé par la succession d'opérations suivantes :

1- Préparation à l'aide d'une spatule d'un mélange de deux composants, respectivement du polydiméthylsiloxane (PDMS) et un agent de réticulation selon un ratio de 10/1 (ces composants étant disponibles auprès de Dow-Corning sous la dénomination de SylGard 184) ;

2- Coulée de ce mélange dans un récipient en plexiglas sur une hauteur de 1,5 mm épaisseur;

3- Réticulation du mélange ainsi coulé pendant 2 heures à 80°C;

4- Placement du modèle mentionné ci-dessus sur le mélange ainsi réticulé ;

5- Coulée du mélange défini au point 1 ci-dessus sur le modèle jusqu'à le recouvrir complètement;

6- Réticulation du mélange ainsi coulé pendant 2 heures à 80°C ;

7- Démoulage manuel du récipient en plexiglas du moule ainsi obtenu;

8- Ouverture du moule en PDMS en deux parties à l'aide d'un scalpel afin de retirer le modèle de départ pris dans le moule en PDMS.

Les deux parties du moule en PDMS sont ensuite recollées après activation par plasma selon les conditions suivantes :

1- On place les deux parties du moule dans un plasma $O_2$ (Plasma $O_2$ AST Product Inc), les conditions suivantes étant appliquées pour activer les fonctions de surface du PDMS ($PO_2$ 1 bar ; Puissance 100 Watt; Durée 1 minute ; Réseau d'adaptation 50-50% ; Gaz 120 ; Flux de gaz 60 ; Point de fonctionnement 0,5) ;

2- Après application du plasma, les deux surfaces du moule à coller sont mises en contact. Une pression est exercée pour améliorer le contact entre les deux surfaces et améliorer ainsi le collage

Les deux parties du moule sont ainsi collées, moyennant quoi l'on obtient un moule présentant une cavité interne correspondant à la forme de l'objet à mouler.

b) Etape de la préparation de la solution sol-gel

La solution sol-gel est préparée par la succession suivante d'opérations :

1- Préparation d'une solution 1 : Mélange à température ambiante pendant 4 heures sous agitation de 0,885 mL de tétraméthylorthosilicate (obtenu auprès du fournisseur Sigma-Aldrich), de 2,5 mL de méthanol et 0,432 mL d'eau, auxquels on ajoute 4 mL d'éthanol anhydre puis 4 mL d'acide chlorhydrique à 1M sous agitation ;

2- Préparation d'une solution 2 : Mélange à température ambiante pendant 4 heures sous agitation de 0,75 mL de n-propoxide de zirconium et d'acide acétique (0,25 mL) ;

3- Mélange à -20°C de la solution 1 et de la solution 2 et ajout de 0,5 mL de 4-aminobutyltriéthoxysilane (obtenu auprès du fournisseur ABCR)

c) Fabrication du rétroréflecteur en tant que tel

La solution sol-gel obtenue à l'étape b) est ensuite introduite dans le moule défini à l'étape a). Cette solution gélifie en environ 2 minutes.

Le moule est alors placé dans une étuve à 50°C pendant 6 jours pour le séchage du gel, à l'issue le gel est transformé en xérogel.

A l'issue du séchage, un rétreint d'un facteur 2 est observé dans toutes les dimensions.

La figure 2 représente une photographie de l'objet obtenu (partie b)) à côté de son modèle (partie a)).

d) Caractérisation du xérogel constitutif du rétroréflecteur

Dans un premier temps, il est procédé à la détermination de la porosité du xérogel. De manière précise, cette porosité est déterminée par adsorption-désorption d'azote à 77K.

La surface spécifique développée est de 421 $m^2/g$ et le volume poreux est de 0,27 $cm^3/g$.

Etant donné que la densité mesurée du xérogel est de 0,71 $g/cm^3$, la porosité est de 0,38.

Dans un deuxième temps, il est procédé à la mesure de l'indice de réfraction. Cette mesure est effectuée à l'aide d'un microscope.

Pour ce faire, on pose sur une lame de verre un parallélépipède de dimensions 2 mm*5 mm*10 mm, lequel est en xérogel réalisé dans les mêmes conditions que celui constitutif du rétroréflecteur.

Cette lame en verre est ensuite disposée sur la platine d'un microscope.

Puis, on réalise la mise au point sur la face supérieure de la lame de verre et on relève la valeur lue sur le vernier de profondeur, cette valeur étant identifiée ci-après par l'indice $z_0$.

Ensuite, on réalise la mise au point sur l'image de la lame de verre à travers le xérogel et on relève la valeur lue sur le vernier de profondeur, cette valeur étant identifiée ci-après par l'indice $z_1$.

Enfin, on réalise la mise au point sur la face supérieure du xérogel et on relève la valeur lue sur le vernier de profondeur, cette valeur étant identifiée ci-après par l'indice $z_2$.

L'indice de réfraction est obtenu par l'équation suivante :

$$n' = (z_2 - z_0)/(z_2 - z_1)$$

Cette équation peut s'expliquer, de manière théorique, sur la base de la figure 3, illustrant le schéma optique de la mesure d'indice optique à l'aide d'un microscope, ce schéma illustrant la marche des rayons dans le microscope et un axe vertical reportant les valeurs $z_0$, $z_1$ et $z_2$.

A partir de ce schéma, les relations suivantes sont établies :

$$\tan i1 = \frac{x}{z2 - z1}$$

$$\tan i2 = \frac{x}{z2 - z0}$$

D'après la loi de Descartes (réfraction sur un dioptre plan), on a :

$$n' = \frac{\sin i1}{\sin i2}$$

Pour des angles $i_1$ et $i_2$ petits, il est possible de développer au premier ordre :

$$\tan i = i + o(i)$$

$$\sin i = i + o(i)$$

Il est alors possible d'exprimer l'indice de réfraction n' du xérogel en fonction de $z_0$, $z_1$ et $z_2$ :

$$n' = \frac{\sin i1}{\sin i2} \approx \frac{\tan i1}{\tan i2}$$

$$n' = \frac{z2 - z0}{z2 - z1}$$

Enfin, dans un troisième temps, il est procédé à un essai pour observer l'effet de rétroréflexion produit par le rétroréflecteur réalisé à l'étape c).

Pour ce faire, comme illustré sur la figure 4, on interpose entre une source lumineuse 9 et le rétroréflecteur 11, une lame semi-réfléchissante 13, qui va permettre de diriger le rayon rétroréfléchi vers un dispositif de caméra 15, qui va enregistrer l'image issue de la rétroréflexion.

Cette image est reproduite au niveau de la figure 5.

EXEMPLE 2

Cet exemple a pour visée de démontrer l'efficacité du rétroréflecteur préparé à l'exemple 1 pour la détection d'un composé organique volatif (COV), qui est l'orthonitrophénol (ce composé étant ici obtenu auprès du fournisseur Sigma Aldrich), lequel répond à la formule suivante :

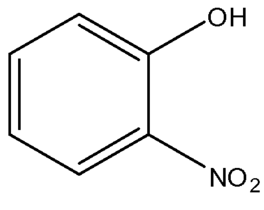

Ce composé peut être émis après hydrolyse d'un substrat enzymatique par une enzyme spécifique d'un microorganisme. La détection d'un tel composé peut

présenter un intérêt certain pour la détection indirecte de microorganismes, comme illustré par l'article Phys.Chem.Chem.Phys, vol.15, n°38, p.15840-15844.

Le rétroréflecteur (référence 17) préparé à l'exemple 1 est placé dans un septum en caoutchouc (référence 19), qui ferme hermétiquement un flacon (référence 21) d'un volume de 15 mL, comme illustré sur la figure 6.

Pour ce faire, le septum est percé à l'emporte-pièce, afin d'obtenir un trou de 6 mm de diamètre, lequel est destiné à accueillir le rétroréflecteur. Une fois placé sur le flacon via le septum, le rétroréflecteur présente une extrémité anguleuse dirigée vers l'intérieur du flacon.

Dans le flacon, avant le positionnement du septum, on introduit initialement 5 mL d'une solution aqueuse d'acide 2-(N-morpholino)éthanesulfonique (obtenu auprès du fournisseur Sigma Aldrich), cette solution présentant un pH de 6,1.

Pour être plus précis, l'orthonitrophénol se présente sous deux formes : une forme protonée à un pH inférieur à 7,2 et une forme déprotonée à un pH supérieur à 7,2. Or seule la forme protonée est volatile, ce qui explique la raison pour laquelle il est nécessaire de tamponner la solution à un pH inférieur à 7,2, afin d'être en présence de la forme volatile de l'orthonitrophénol.

A t=0 seconde, le flacon est fermé avec le septum équipé du rétroréflecteur. A t=900 secondes, on introduit ensuite 5 $\mu$L d'orthonitrophénol à 0,1 mol/L avec une seringue traversant le septum en caoutchouc, moyennant il résulte une solution présentant une concentration finale de 100 $\mu$lmol/L.

Une fraction de l'orthonitrophénol ajouté passe en phase gazeuse, du fait du phénomène de protonation se produisant dans le flacon. Le rétroréflecteur capte une partie du gaz émis. La présence dans le rétroréflecteur de groupe amines issus du précurseur 4-aminobutyltriéthoxysilane permet de déprotoner l'orthonitrophénol.

La forme déprotonée de l'orthonitrophénol a pour propriété d'absorber à la longueur d'onde de 415 nm ($\varepsilon$=3500 $L.mol^{-1}.cm^{-1}$).

Afin de suivre la variation d'absorption du rétroréflecteur à 415 nm, le montage est éclairé avec deux diodes électroluminescentes : respectivement, une diode émettant à 415 nm et une diode émettant à 590 nm, qui sert de référence.

Les intensités de ces deux longueurs d'onde sont mesurées grâce à une caméra couleur en séparant les canaux rouge-vert-bleu, les intensités étant intégrées sur une région d'intérêt correspondant à un disque, qui couvre l'image du rétroréflecteur. L'intensité à 415 nm est mesurée sur le canal bleu alors que l'intensité à 590 nm est mesurée sur le canal rouge.

L'évolution de l'intensité à 415 nm et à 590 nm en fonction du temps (en minutes) est illustré par la figure 7 (courbe a) pour l'intensité à 415 nm et courbe b) pour l'intensité à 590 nm).

Pour l'intensité à 590 nm (signal de référence), on observe une diminution continue de l'intensité.

Pour l'intensité à 415 nm, on observe aisément que le signal décroche à 15 minutes, ce qui correspond au moment de l'injection de l'orthonitrophénol dans le flacon. Ceci atteste donc bien de la capacité des rétroréflecteurs de l'invention à piéger l'orthonitrophénol sous forme déprotonée.

## Revendications

1. Rétroréflecteur apte à être placé au contact d'un environnement, comprenant, comme matériau constitutif, c'est-à-dire le matériau compris entre les faces du rétroréflecteur et délimitant également ces dernières, un matériau poreux capteur d'un paramètre dudit environnement, lequel paramètre dudit environnement est un gaz, ledit matériau induisant une modification des propriétés de transmission optique du rétroréflecteur en présence dudit paramètre et ledit rétroréflecteur étant apte à recevoir un faisceau lumineux incident par une première face et à réémettre un faisceau lumineux par ladite première face.

2. Rétroréflecteur selon la revendication 1, qui comprend trois premières faces, dites faces arrières, définissant un trièdre, lesdites trois faces étant convergentes en un point formant un sommet du trièdre, et une quatrième face, dite face avant, opposée audit sommet du trièdre et refermant ledit trièdre, ledit matériau s'étendant entre ces faces.

3. Rétroréfleeteur selon la revendication 2, dans lequel chaque face arrière s'étend respectivement selon un premier plan, un deuxième plan et un troisième plan , lesdits plans étant sensiblement orthogonaux deux à deux, le faisceau lumineux ressortant selon une direction sensiblement parallèle à celle du faisceau lumineux incident.

4. Rétroréflecteur selon l'une quelconque des revendications précédentes, qui présente une forme de coin de cube.

5. Rétroréflecteur selon l'une quelconque des revendications précédentes, dans lequel le matériau poreux est un matériau à porosité ouverte.

6. Rétroréflecteur selon l'une quelconque des revendications précédentes, qui présente un indice de réfraction allant de 1,2 à 2.

7. Rétroréflecteur selon l'une quelconque des revendications 1 à 5, qui présente un indice de réfraction allant de 1,66 à 2,7.

8. Rétroréflecteur selon l'une quelconque des revendications précédentes, dans lequel le matériau capteur est un matériau organique ou inorganique, comprenant, en son sein, un ou plusieurs composés porteurs d'un ou plusieurs groupes remplissant une fonction de captation d'un paramètre de l'environnement.

**9.** Rétroréflecteur selon la revendication 8, dans lequel, lorsque le matériau inorganique est un matériau du type oxyde(s) inorganique(s), le matériau du type oxyde(s) inorganique(s) comprend un ou plusieurs oxydes d'un élément choisi parmi le silicium, le titane, le zirconium, l'aluminium, le vanadium, le chrome, l'yttrium, le tungstène, le niobium, le molybdène.

**10.** Rétroréflecteur selon la revendication 8 ou, 9, dans lequel le ou les composés porteurs d'un ou plusieurs groupes sont inclus dans le matériau organique ou inorganique sans être liés de manière covalente à ce dernier.

**11.** Rétroréflecteur selon la revendication 8 ou 9, dans lequel le ou les composés porteurs d'un ou plusieurs groupes sont liés, de manière covalente, audit matériau organique ou inorganique.

**12.** Rétroréflecteur selon l'une quelconque des revendications 8 à 11, dans lequel le ou les composés porteurs d'un ou plusieurs groupes remplissant une fonction de captation d'un paramètre de l'environnement présentent un ou plusieurs groupes aptes à capter ledit composé chimique avec modification chimique de celui-ci et/ou aptes à capter ledit composé chimique par affinité chimique sans modification chimique du composé chimique.

**13.** Procédé de détection d'au moins un paramètre de l'environnement, qui est un gaz, comprenant les étapes suivantes :

- une étape de mise en contact d'un rétroréflecteur tel que défini selon l'une quelconque des revendications 1 à 12 avec un environnement, dont on veut détecter un gaz donné ;
- une étape d'éclairage du rétroréflecteur par un premier faisceau lumineux présentant une première longueur d'onde, ladite première longueur d'onde correspondant à une longueur d'onde susceptible de provoquer une variation de la transmission optique du matériau constituant le rétroréflecteur après captation du paramètre de l'environnement ;
- une étape d'analyse d'un premier faisceau lumineux réfléchi par le rétroréflecteur, de laquelle l'on en déduit, le cas échéant, la présence dudit paramètre de l'environnement.

**14.** Procédé de détection selon la revendication 13, comprenant, en outre, les étapes suivantes :

- une deuxième étape d'éclairage du rétroréflecteur par le faisceau lumineux comprenant une deuxième longueur d'onde, ladite deuxième longueur d'onde correspondant à une longueur d'onde susceptible de provoquer une deuxième variation de la transmission optique du matériau constituant le rétroréflecteur après captation du paramètre de l'environnement, ladite deuxième variation étant moindre que ladite première variation ;
- une étape d'analyse d'un deuxième faisceau lumineux réfléchi par le rétroréflecteur ;
- une étape de comparaison desdits premier et deuxième faisceaux réfléchis, de laquelle l'on en déduit, le cas échéant, la présence du paramètre de l'environnement.

**15.** Procédé de détection selon la revendication 14, dans lequel :

- le premier faisceau lumineux comporte une pluralité de longueurs d'onde ;
- l'étape d'analysé du faisceau réfléchi comprend une analyse dudit faisceau à au moins deux longueurs d'onde de ladite pluralité de longueurs d'onde.

**Patentansprüche**

**1.** Rückstrahler, der in Kontakt mit einer Umgebung gebracht werden kann, umfassend als ein konstitutives Material, d. h. das zwischen den Flächen des Rückstrahlers enthaltene und diese letzteren gleichermaßen begrenzende Material, ein poröses Material ist, das ein Sensor für einen Parameters der Umgebung ist, wobei der Parameter der Umgebung ein Gas ist, wobei das Material eine Modifikation der optischen Transmissionseigenschaften des Rückstrahlers in Gegenwart des Parameters induziert und der Rückstrahler in der Lage ist, einen über eine erste Fläche einfallenden Lichtstrahl zu empfangen und einen Lichtstrahl von der ersten Fläche zu emittieren.

**2.** Rückstrahler nach Anspruch 1, der drei erste Flächen umfasst, Rückseiten genannt, die ein Dreieck definieren, wobei die drei Flächen an einem Punkt zusammenlaufen, der einen Scheitelpunkt des Dreieck bildet, und eine vierte Fläche, Vorderfläche genannt, die dem Scheitelpunkt des Dreiecks gegenüberliegt, und das Dreieck schließt, wobei sich das Material zwischen diesen Flächen erstreckt.

**3.** Rückstrahler nach Anspruch 2, bei welchem sich jede Rückseite jeweils in einer ersten Ebene, einer zweiten Ebene und einer dritten Ebene erstreckt, wobei die Ebenen paarweise im Wesentlichen orthogonal zueinander verlaufen und der Lichtstrahl in einer Richtung austritt, die im Wesentlichen parallel zu der des einfallenden Lichtstrahls verläuft.

**4.** Rückstrahler nach einem der vorhergehenden Ansprüche, der die Form einer Würfelecke aufweist.

**5.** Rückstrahler nach einem der vorstehenden Ansprüche, bei welchem das poröse Material ein offenporiges Material ist.

**6.** Rückstrahler nach einem der vorhergehenden Ansprüche, der einen Brechungsindex im Bereich von 1,2 bis 2 aufweist.

**7.** Rückstrahler nach einem der Ansprüche 1 bis 5, der einen Brechungsindex im Bereich von 1,66 bis 2,7 aufweist.

**8.** Rückstrahler nach einem der vorstehenden Ansprüche, bei welchem das Sensormaterial ein organisches oder anorganisches Material ist, das in sich eine oder mehrere Verbindungen umfasst, die eine oder mehrere Gruppen tragen, die eine Funktion zum Erfassen eines Parameters der Umgebung erfüllen.

**9.** Rückstrahler nach Anspruch 8, bei welchem dann, wenn das anorganische Material ein anorganisches Oxidmaterial ist, das anorganische Oxidmaterial ein oder mehrere Oxide eines Elements umfasst, ausgewählt aus Silizium, Titan, Zirkonium, Aluminium, Vanadium, Chrom, Yttrium, Wolfram, Niob, Molybdän.

**10.** Rückstrahler nach Anspruch 8 oder 9, bei welchem die Trägerverbindung(en) einer oder mehrerer Gruppen in dem organischen oder anorganischen Material enthalten sind, ohne kovalent daran gebunden zu sein.

**11.** Rückstrahler nach Anspruch 8 oder 9, bei welchem die Trägerverbindung(en) einer oder mehrerer Gruppen kovalent an das organische oder anorganische Material gebunden sind.

**12.** Rückstrahler nach einem der Ansprüche 8 bis 11, bei welchem die Verbindung oder die Verbindungen, die eine oder mehrere Gruppen tragen, die eine Funktion zum Erfassen eines Parameters der Umgebung erfüllen, eine oder mehrere Gruppen aufweisen, die in der Lage sind, die chemische Verbindung mit ihrer chemischen Modifikation zu erfassen und/oder die chemische Verbindung durch chemische Affinität ohne chemische Modifikation der chemischen Verbindung zu erfassen.

**13.** Verfahren zum Detektieren mindestens eines Umgebungsparameters, der ein Gas ist, umfassend die folgenden Schritte:

- einen Schritt des In-Kontakt-Bringens eines Rückstrahlers nach einem der Ansprüche 1 bis 12 mit einer Umgebung, aus der ein bestimmtes Gas nachgewiesen werden soll;
- einen Schritt des Beleuchtens des Rückstrahlers mit einem ersten Lichtstrahl einer ersten Wellenlänge, wobei die erste Wellenlänge einer Wellenlänge entspricht, die geeignet ist, eine Variation in der optischen Transmission des den Rückstrahler bildenden Materials nach dem Erfassen des Umgebungsparameters zu bewirken;
- einen Schritt des Analysierens eines ersten Lichtstrahls, der von dem Rückstrahler reflektiert wird, aus dem das Vorhandensein des genannten Umgebungsparameters, gegebenenfalls, abgeleitet werden kann.

**14.** Detektionsverfahren nach Anspruch 13, ferner umfassend die folgenden Schritte:

- einen zweiten Schritt des Beleuchtens des Rückstrahlers durch den Lichtstrahl, der eine zweite Wellenlänge umfasst, wobei die zweite Wellenlänge einer Wellenlänge entspricht, die nach dem Erfassen des Umgebungsparameters eine zweite Variation in der optischen Transmission des Materials, das den Rückstrahler bildet, verursachen kann, wobei die zweite Variation kleiner als die erste Variation ist;
- einen Schritt des Analysierens eines zweiten Lichtstrahls, der von dem Rückstrahler reflektiert wird;
- einen Schritt des Vergleichs der ersten und zweiten reflektierten Strahlen, aus dem, gegebenenfalls, das Vorhandensein des Umgebungsparameters abgeleitet wird.

**15.** Detektionsverfahren nach Anspruch 14, wobei:

- der erste Lichtstrahl eine Vielzahl von Wellenlängen aufweist;
- der Schritt des Analysierens des reflektierten Strahls eine Analyse des reflektierten Strahls bei mindestens zwei Wellenlängen der Vielzahl von Wellenlängen.

## Claims

**1.** A retroreflector capable of being placed in contact with an environment, comprising, as a constitutive material, i.e. the material comprised between the faces of the retroreflector and also delimiting the latter, a porous material capturing a parameter of said environment, said parameter of said environment being a gas, said material inducing a modification of the optical transmission properties of the retroreflector, in the presence of said parameter and said retroreflector being able to receive an incident light beam through a first face and of re-emitting a light beam through said first face.

**2.** The retroreflector according to claim 1, which comprises three first faces, so called rear faces, defining a trihedron, said three faces being convergent in a point forming an apex of the trihedron, and a fourth face, a so called opposite front face to said apex of the trihedron, and containing said trihedron, said material extending between these faces.

**3.** The retroreflector according to claim 2, wherein each rear face respectively extends along a first plane, a second plane and a third plane, said planes being substantially orthogonal with each other, the light beam emerging along a direction substantially parallel to that of the incident light beam.

**4.** The retroreflector according to any of the preceding claims, which has a cube corner shape.

**5.** The retroreflector according to any of the preceding claims, wherein the porous material is a material with open porosity.

**6.** The retroreflector according to any of the preceding claims, which has a refractive index ranging from 1.2 to 2.

**7.** The retroreflector according to any of claims 1 to 5, which has a refractive index ranging from 1.66 to 2.7.

**8.** The retroreflector according to any of the preceding claims, wherein the capture material is an organic or inorganic material, comprising, inside it, one or several compounds bearing one or several groups fulfilling a function for capturing a parameter of the environment.

**9.** The retroreflector according to claim 8, wherein, when the inorganic material is a material of the inorganic oxide(s) type, the material of the inorganic oxide(s) type comprises one or several oxides of an element selected from among silicon, titanium, zirconium, aluminium, vanadium, chromium, yttrium, tungsten, niobium, molybdenum.

**10.** The retroreflector according to claim 8 or 9, wherein the compound(s) bearing one or several groups are included in the organic or inorganic material without being covalently bound to the latter.

**11.** The retroreflector according to claim 8 or 9, wherein the compound(s) bearing one or several groups are covalently bound to said organic or inorganic material.

**12.** The retroreflector according to any of claims 8 to 11, wherein the compound(s) bearing one or several groups fulfilling a function for capturing a parameter of the environment have one or several groups able to capture said chemical compound with chemical modification of the latter and/or able to capture said chemical compound by chemical affinity without any chemical modification of the chemical compound.

**13.** A method for detecting at least one parameter of the environment, which is a gas, comprising the following steps:

- a step for putting into contact a retroreflector as defined according to any of claims 1 to 12 with an environment, for which the intention is to detect a given gas;
- a step for illuminating the retroreflector with a first light beam having a first wavelength, said first wavelength corresponding to a wavelength which may cause a variation in the optical transmission of the material making up the retroreflector after capturing the parameter of the environment;
- a step for analyzing a first light beam reflected by the retroreflector, from which is inferred, if necessary, the presence of said parameter of the environment.

**14.** The detection method according to claim 13, further comprising the following steps:

- a second step for illuminating the retroreflector with the light beam comprising a second wavelength, said second wavelength corresponding to a wavelength which may cause a second variation of the optical transmission of the material making up the retroreflector after capturing the parameter of the environment, said second variation being less than said first variation;
- a step for analyzing a second light beam reflected by the retroreflector;
- a step for comparing said first and second reflected beams, from which is inferred, if necessary, the presence of the parameter of the environment.

**15.** The detection method according to claim 14, wherein:

- the first light beam includes a plurality of wavelengths;
- the step for analyzing the reflected beam comprises an analysis of said beam at at least two

wavelengths from said plurality of wavelengths.

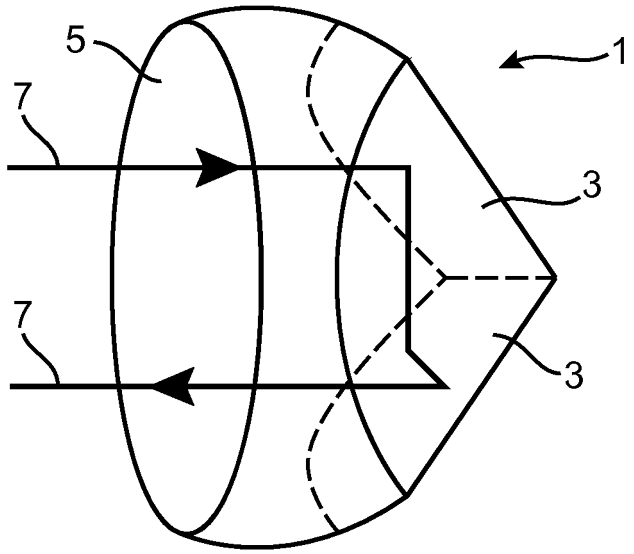


FIG.1

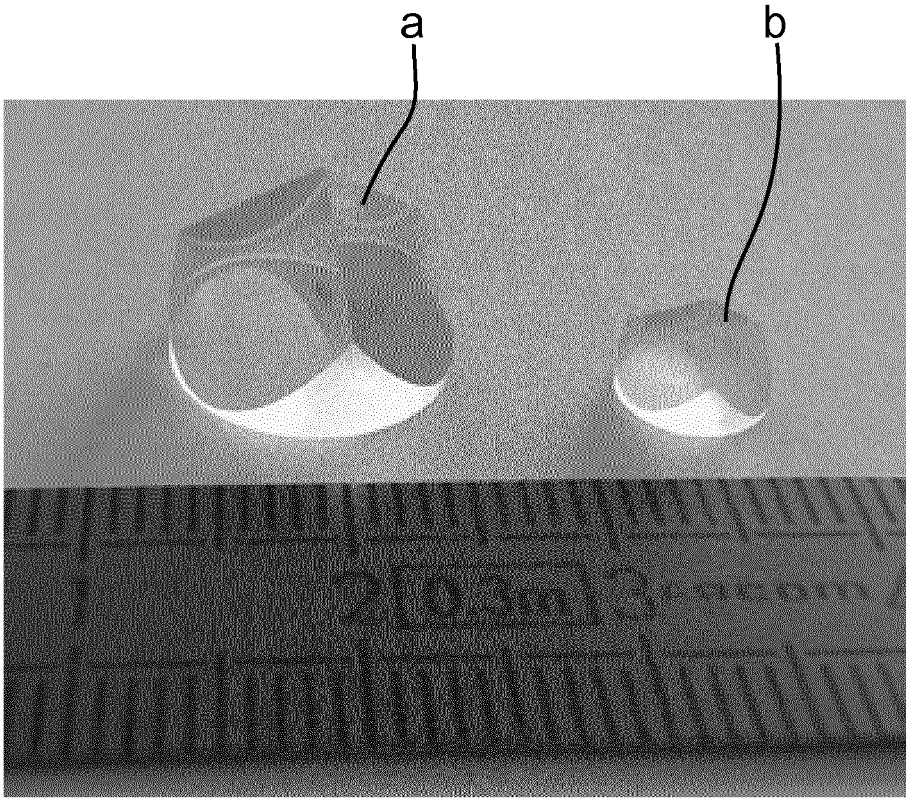


FIG.2

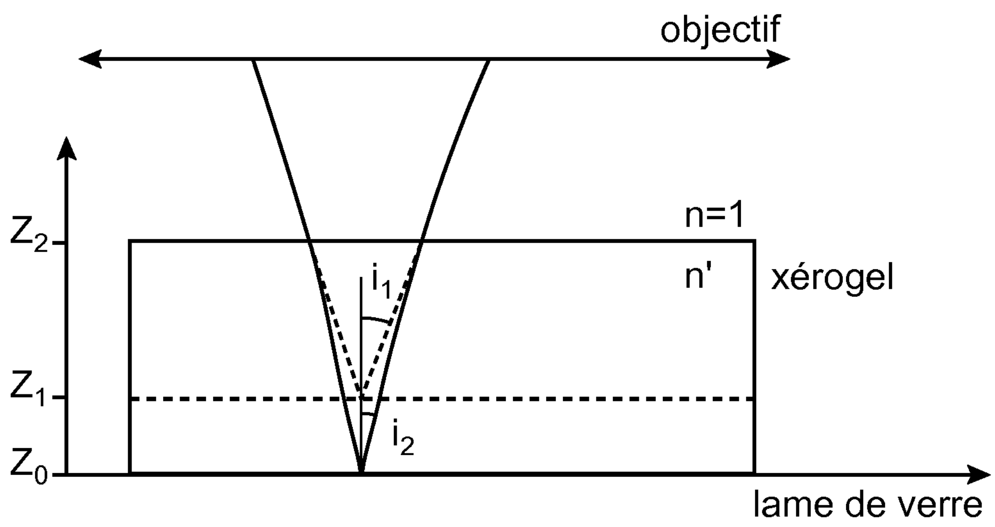


FIG.3

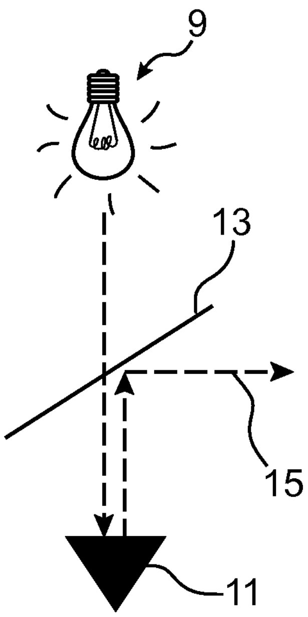


FIG.4

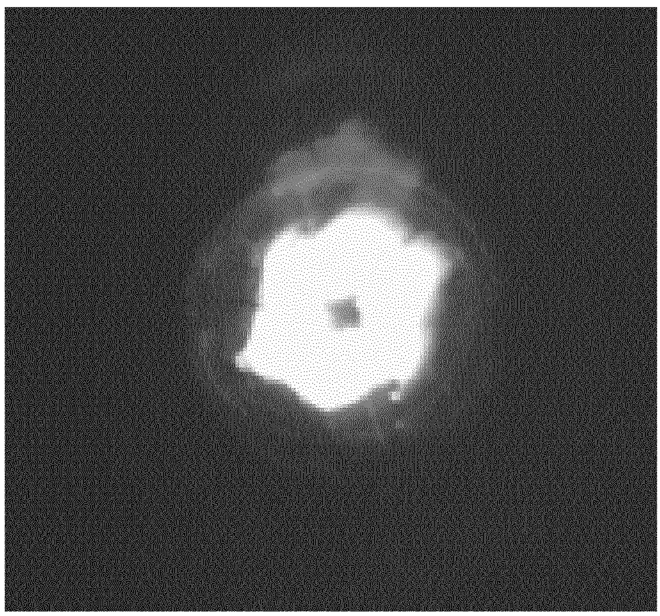

FIG.5

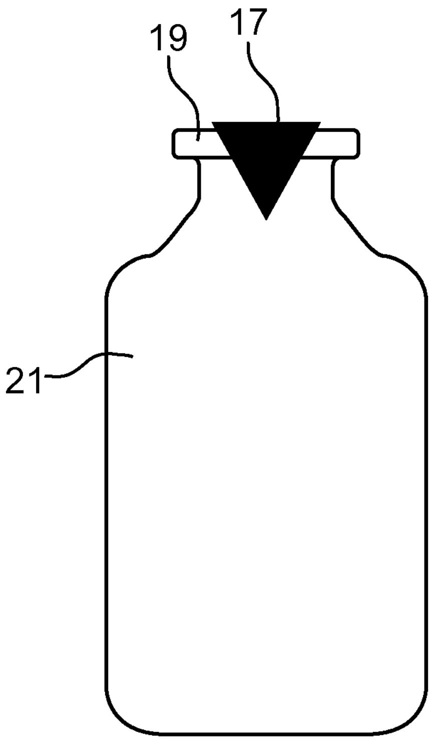
19
17
21

FIG.6

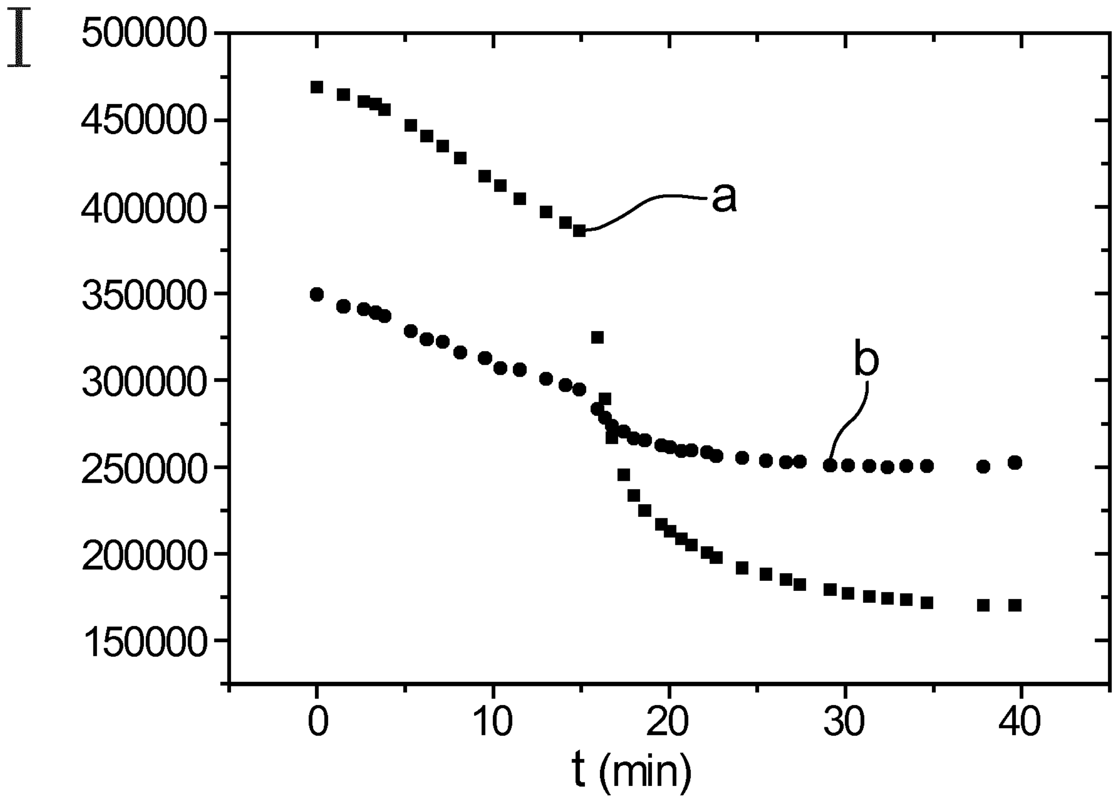
I
500000
450000
400000
350000
300000
250000
200000
150000
a
b
0
10
20
30
40
t (min)

FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20120140224 A
- US 20070036680 A
- US 2012140224 A1
- US 2006088946 A1
- US 2003203212 A1
- US 2006285116 A1
- WO 2014154712 A1
- FR 2980789


### Littérature non-brevet citée dans la description


- *Proceedings of SPIE-The International Society for Optical Engineering 2839,* 203-214
- *Phys.Chem.Chem.Phys,* vol. 15 (38), 15840-15844